# EUROPEAN PATENT APPLICATION

(11) **EP 4 847 085 A1**
(43) Date of publication of application: **02.12.2026**
(21) Application number: 24920600.4
(22) Date of filing: 23.12.2024
(51) Int. Cl.: A61N 5/10

(54) **PHANTOM USED FOR VERIFYING RADIATION DOSE FOR TREATMENT AND METHOD FOR VERIFYING RADIATION DOSE BY USING SAME**

(30) Priority: 22.01.2024 KR 20240009226
(71) Applicant: National Cancer Center, Goyang-si, Gyeonggi-do 10408 (KR); Vieworks Co., Ltd., Anyang-si, Gyeonggi-do 14055 (KR)
(72) Inventor: KIM, Hak Soo, Goyang-si, Gyeonggi-do 10371 (KR); KIM, Doo Ho, Anyang-si, Gyeonggi-do 14055 (KR); KIM, Chan Kyu, Goyang-si, Gyeonggi-do 10450 (KR); YANG, Han Soo, Anyang-si, Gyeonggi-do 14055 (KR); HAN, Young Jun, Anyang-si, Gyeonggi-do 14055 (KR); SHIN, Dong Ho, Suwon-si, Gyeonggi-do 16295 (KR); YANG, Hye Jeong, Seoul 06988 (KR); LEE, Se Byeong, Goyang-si, Gyeonggi-do 10375 (KR); LIM, Young Kyung, Paju-si, Gyeonggi-do 10903 (KR); JEONG, Jong Hwi, Seoul 05536 (KR)
(74) Representative: Habermann, Hruschka & Schnabel
(86) International application number: PCT/KR2024/020919
(87) International publication number: WO 2025/159351

(57) **Abstract**

The present invention relates to a phantom used for verifying a radiation dose for treatment and a method for verifying a radiation dose by using same. The phantom according to the present invention comprises: an outer case forming an inner space; and multiple detection points for detecting the dose of incident radiation. The multiple detection points comprise: a radiation detection panel three-dimensionally arranged in the inner space; and a driving circuit board positioned in the inner space and connected to the radiation detection panel.

## Description

### Technical Field

The present disclosure relates to a phantom used for verifying a radiation dose for treatment and a method for verifying a radiation dose by using the same.

### Background Art

Various radiation treatment technologies have been introduced, and accurate treatment is required in order to deliver a minimum dose to normal tissues around a tumor and accurately deliver a prescribed radiation dose to the tumor by calculating a precise and optimized dose distribution.

For such accurate treatment, it is important to verify a calculated radiation dose before treatment. For this purpose, various dose measurement devices have been used, but such devices are insufficient for verifying a three-dimensional radiation dose.

### Disclosure

### Technical Problem

Accordingly, an object of the present disclosure is to provide a phantom used for verifying a radiation dose for treatment and a method for verifying a radiation dose by using the same.

### Technical Solution

The object of the present disclosure is achieved by a phantom used for verifying a radiation dose for treatment, the phantom including: an outer case forming an inner space; and multiple detection points for detecting a dose of incident radiation, in which the multiple detection points include a radiation detection panel three-dimensionally arranged in the inner space and a driving circuit board positioned in the inner space and connected to the radiation detection panel.

The radiation detection panel may be flexible, and the radiation detection panel may be rolled in a cylindrical shape or rolled in a spiral shape.

The radiation detection panel may include an electrical insulating layer having an upper surface and a lower surface, an upper electrode on the upper surface of the electrical insulating layer, and a plurality of pixel units electrically connected to the electrical insulating layer and directly contacting the lower surface of the electrical insulating layer.

The radiation detection panel may include a first radiation detection panel rolled in a cylindrical shape, and a second radiation detection panel rolled in a cylindrical shape and positioned within the first radiation detection panel.

The phantom may further include: an inner cover positioned between the first radiation detection panel and the second radiation detection panel, in which the inner cover prevents interference between the first radiation detection panel and the second radiation detection panel.

The phantom may further include: an integrated circuit connecting the radiation detection panel and the circuit board, in which the circuit board is connected to an external power line and signal line.

The radiation detection panel may include a first radiation detection panel having a flat plate shape, and a second radiation detection panel spaced apart from the first radiation detection panel and having a width different from that of the first radiation detection panel.

The inner space may have a cylindrical shape.

The object of the present disclosure is achieved by a method for verifying a radiation dose, the method including: irradiating radiation onto a phantom including a radiation detection panel; and analyzing the radiation dose using the radiation detected by the radiation detection panel, in which the radiation detection panel includes multiple detection points for detecting a dose of incident radiation, and the multiple detection points are three-dimensionally arranged in the inner space.

The phantom may form a cylindrical inner space, the radiation detection panel may be positioned in the inner space, and the radiation may be irradiated 360 degrees along a circumference of the inner space.

The radiation detection panel may be flexible, and the radiation detection panel may be rolled in a cylindrical shape or rolled in a spiral shape.

The radiation detection panel may include a first radiation detection panel rolled in a cylindrical shape, and a second radiation detection panel rolled in a cylindrical shape and positioned within the first radiation detection panel.

The radiation may include at least one of X-rays and proton beams.

The verification method may be used for verifying a radiation dose of volumetric intensity modulated rotational treatment.

### Advantageous Effects

According to the present disclosure, there are provided a phantom used for verifying a radiation dose for treatment and a method for verifying a radiation dose by using the same.

### Description of Drawings

FIG. 1 is a perspective view of a phantom according to a first embodiment of the present disclosure,
FIG. 2 is an exploded perspective view of the phantom according to the first embodiment of the present disclosure,
FIG. 3 illustrates a radiation detection panel and an integrated circuit in the phantom according to the first embodiment of the present disclosure,
FIG. 4 illustrates a cross-sectional view taken along line IV-IV' of FIG. 1,
FIG. 5 illustrates a measurement principle of a flexible panel in the phantom according to the first embodiment of the present disclosure,
FIG. 6 is an exploded perspective view of a phantom according to a second embodiment of the present disclosure,
FIG. 7 is a cross-sectional view of the phantom according to the second embodiment of the present disclosure,
FIG. 8 is a cross-sectional view of a phantom according to a third embodiment of the present disclosure,
FIG. 9 is a cross-sectional view of a phantom according to a fourth embodiment of the present disclosure, and
FIGS. 10 and 11 illustrate a method for verifying a radiation dose using the phantom of the present disclosure.

### Mode for Invention

Hereinafter, the present disclosure will be described in more detail with reference to the accompanying drawings.

The accompanying drawings are merely examples illustrated for more specifically describing the technical spirit of the present disclosure, and thus the technical spirit of the present disclosure is not limited to the accompanying drawings. In the accompanying drawings, thicknesses, lengths, and the like of respective parts may be exaggerated for convenience of description.

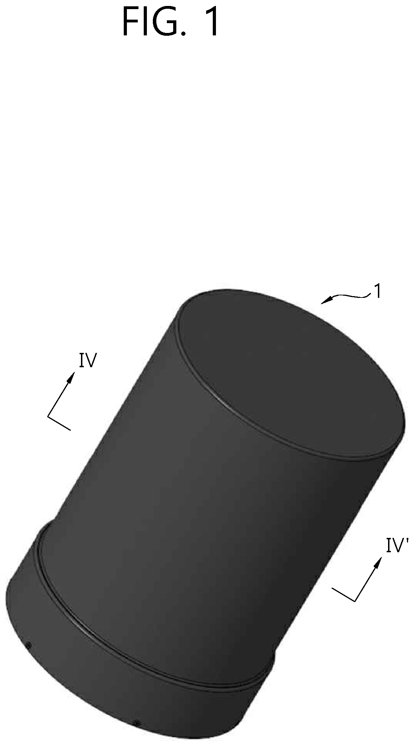

A phantom according to a first embodiment of the present disclosure will be described with reference to FIGS. 1 to 5.

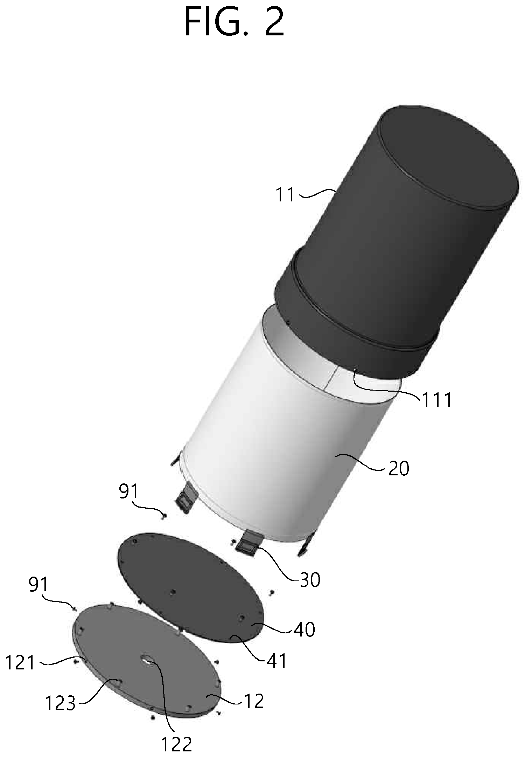

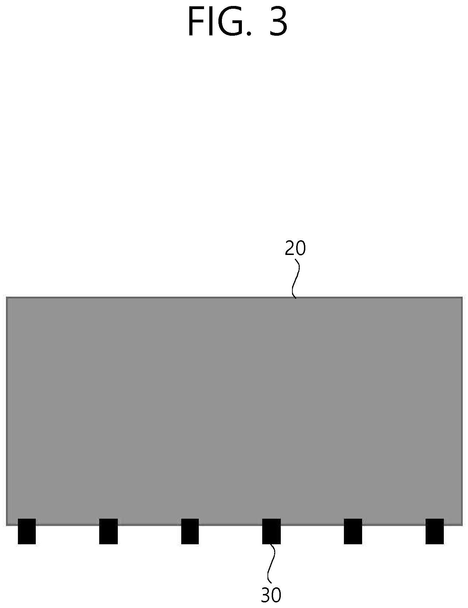

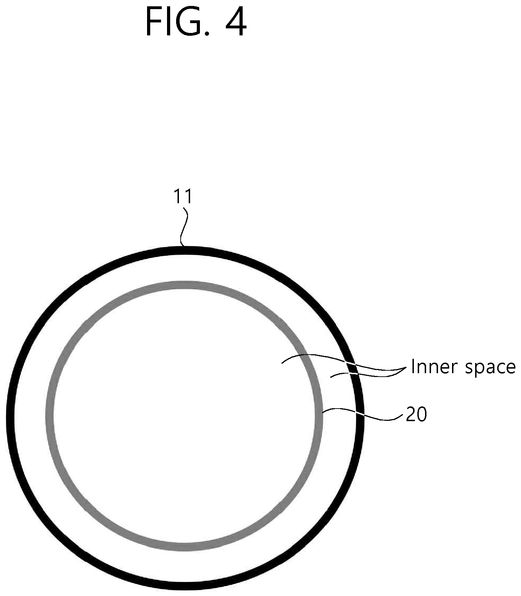

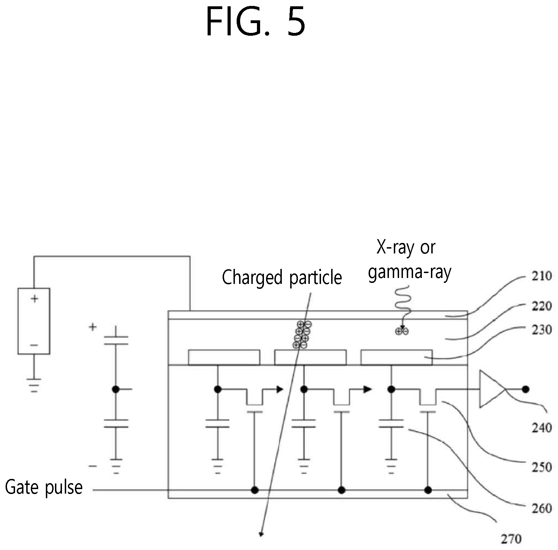

FIG. 1 is a perspective view of a phantom according to a first embodiment of the present disclosure, FIG. 2 is an exploded perspective view of the phantom according to the first embodiment of the present disclosure, FIG. 3 illustrates a radiation detection panel and an integrated circuit in the phantom according to the first embodiment of the present disclosure, FIG. 4 illustrates a cross-sectional view taken along line IV-IV' of FIG. 1, and FIG. 5 illustrates a measurement principle of a flexible panel in the phantom according to the first embodiment of the present disclosure.

The phantom 1 includes cases 11 and 12, a radiation detection panel 20, an integrated circuit 30, and a circuit board 40.

The phantom 1 has an overall cylindrical shape and extends longitudinally in one direction. In another embodiment, the phantom 1 may have various shapes such as a rectangular pillar shape.

The cases 11 and 12 form an exterior appearance of the phantom 1, and include a body case 11 and a lower case 12. The cases 11 and 12 are formed of thin plates. The cases 11 and 12 may be manufactured of, but are not limited to, reinforced plastic, and in particular may be manufactured of carbon fiber reinforced plastic (CFRP).

Coupling holes 111 and 121 are formed in the cases 11 and 12, a through-hole 122 is formed at a center portion of the lower case 12, and a coupling protrusion 123 protrudes from a circumference of an upper surface of the lower case 12.

The cases 11 and 12 form an inner space, and the inner space has a cylindrical shape. In another embodiment, the inner space may have various shapes such as a rectangular pillar shape.

The radiation detection panel 20, the integrated circuit 30, and the circuit board 40 are accommodated in the inner space. In FIG. 2, diameters and the like of the radiation detection panel 20, the integrated circuit 30, and the circuit board 40 are illustrated as being similar to those of the cases 11 and 12 for convenience.

The radiation detection panel 20 has a flexible characteristic, and has a shape in which a flat plate as illustrated in FIG. 3 is rolled into a cylindrical shape. The radiation detection panel 20 is disposed such that movement thereof is restricted within the inner space.

The radiation detection panel 20 includes multiple detection points for detecting a dose of incident radiation, and the multiple detection points are three-dimensionally arranged in the inner space. In the present embodiment, the detection points are distributed in a cylindrical shape according to the cylindrical state of the radiation detection panel 20.

As illustrated in FIG. 5, the radiation detection panel 20 includes an upper electrode 210, an electrical insulating layer 220, and a charge collection electrode 230, and further includes a charge accumulation amplifier 240, a transistor 250, and a storage capacitor 260. The upper electrode 210 may be formed by sputtering deposition and/or adhesion, and may be formed of any conductive material such as metal. Examples of the electrical insulating layer 220 include, but are not limited to, organic compounds including low-Z materials (for example, chemical elements having a low atomic number of protons in a nucleus), such as hydrogen (H), oxygen (O), carbon (C), nitrogen (N), and fluorine (F), such as polytetrafluoroethylene (TEFLON) and acrylic resin.

Preferred examples of materials for the electrical insulating layer 220 include parylene, BCB (Benzocyclobutene), and polyimide film (KAPTON), which have high dielectric strength. Vacuum deposition may be used for parylene, and adhesion may be used for either parylene or KAPTON by preparing a film tape preferably having a thickness of 50 microns. In FIG. 5, the capacitor represents capacitance of the electrical insulating layer 220 and the storage capacitor 260.

Upon irradiation of radiation onto the phantom 1, the upper electrode 210 is disposed on an upper surface of the electrical insulating layer 220, and the charge collection electrode 230 as a pixel unit is disposed on a lower surface of the electrical insulating layer 220. In one embodiment, the charge collection electrode 230 as the pixel unit is disposed on the lower surface of the electrical insulating layer 220 within the electrical insulating layer 220. In one embodiment, the pixel units are electrically connected to the electrical insulating layer 220, and at least one transistor is connected to each pixel unit such that the pixel units are disposed on the lower surface of the electrical insulating layer 220. Each transistor is connected between one of the charge collection electrodes 230 and ground, and the charge collection electrodes 230 may be positioned within the lower surface of the electrical insulating layer 220.

In another embodiment, the pixel unit may include the charge collection electrode 230, the storage capacitor 260, and the transistor 250. The charge collection electrode 230 collects a charge signal in a pixel region of the electrical insulating layer 220. The storage capacitor 260 is connected to the charge collection electrode 230 to store the charge signal collected by the charge collection electrode 230. The field effect transistor (FET) 250 is connected to the charge collection electrode 230 and acts as a switch between the storage capacitor 260 and the external charge accumulation amplifier 240.

When radiation is applied, at a point in time when charged particles traverse the dielectric layer 220, continuous ionization along a path thereof forms a conductive channel allowing current to be conducted from the upper electrode 210 to the pixel electrode 230. This conductive channel is opened only when the charged particles traverse the dielectric layer 220. Meanwhile, a photo-electric interaction between X-ray or gamma-ray photons and the dielectric layer 220 generates a group of electron-hole pairs localized at an interaction point. Even when there is an electric field between the bias electrode and the pixel electrode, charge separation and mobility of the electron-hole pairs in the dielectric layer 220 are limited. Accordingly, interactions of multiple X-ray or gamma-ray photons at adjacent positions in a pixel are required in order to generate a conductive path allowing current from the bias electrode to the pixel electrode.

A pixel matrix may be arranged in multiple rows and multiple columns such as N rows x M columns. Gate lines of transistors in each row are connected to respective multiple external gate drivers. Data lines of transistors in each column orthogonal to the gate lines are connected to respective multiple charge accumulation amplifiers. Before exposure to a radiation beam, a bias voltage having a magnitude not exceeding a breakdown voltage of the electrical insulator is applied to the upper electrode 210, and a negative voltage is applied to all gate electrodes of the pixel FET transistors. During exposure to the radiation beam, an ionization channel current of the insulating layer 220 increases in proportion to intensity or dose of radiation. Local ionization channel charges on each pixel are stored in the storage capacitor 260.

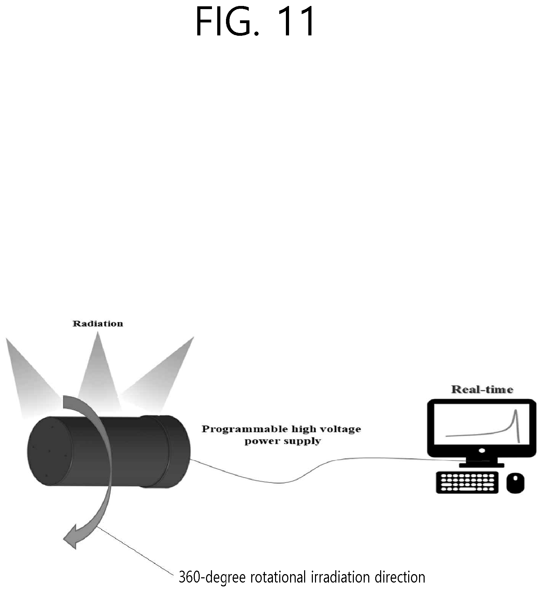

During readout of an image due to exposure to the radiation beam, a gate voltage in one row (row 1, row 2, or row 3) is converted from a negative value to a positive value such that charges stored in respective pixels of the row are transferred to respective charge accumulation amplifiers through orthogonal data lines, and are then digitized by a processing computer (see FIG. 11). After data of one row of the matrix is completed, a potential of the gate line of the row returns to a negative value, and the FET transistors return to an "off" state. A potential of a next row of the gate lines is converted from a negative value to a positive value such that stored charges flow to orthogonal charge accumulation amplifiers. This process is alternately repeated until all charges of the pixel matrix are read.

In one embodiment, the pixel unit array is directly coupled to the electrical insulating layer 220 without an intervening layer for a radiation converter having a charge generation material (CGM) such as amorphous selenium, or without a photoconductive layer for converting X-ray energy into electron-hole pairs. When a thin layer of electrical insulating material without a selenium layer is used, charge generation efficiency due to a photo-electric effect, which is an interaction between photons and the insulating material, is generally 100 times lower than charge generation efficiency due to interaction of photons with selenium. However, when exposed to particle beam radiation, charges passing through the ionization channel maintain high spatial resolution while exhibiting lower-order charge trapping or changes in a local electric field affecting a subsequent image.

In the radiation detection panel 20 described above, each pixel may correspond to a detection point.

In addition, since the radiation detection panel 20 should be flexible, the radiation detection panel 20 may use, for example, a flexible plastic substrate.

The integrated circuit 30 connects the radiation detection panel 20 and the circuit board 40. The integrated circuit 30 may use a readout integrated circuit. The integrated circuit 30 is used as a high-speed multi-channel analog-to-digital converter (ADC) for collecting charges distinguished for respective pixels within the panel. The integrated circuit 30 may include the accumulation amplifier 240 of FIG. 5. The integrated circuit 30 may be included in the circuit board 40, and may be configured separately from the circuit board 40 as illustrated in the drawings in order to maximize a signal-to-noise ratio.

The circuit board 40 has a disk shape, but is not limited thereto. The circuit board 40 is connected to the radiation detection panel 20 through the integrated circuit 30, and is connected to an external power line and signal line (such as a LAN cable) through the through-hole 122. Measurement results of the radiation detection panel 20 are transmitted to the outside through the signal line.

The respective configurations described above are coupled through the coupling holes 41, 111, and 121, coupling members 91 (such as screws), and the coupling protrusion 123, and detailed description of coupling thereof will be omitted.

The inner space of the phantom 1 may be filled with water or solid water. The solid water may use, but is not limited to, an epoxy resin-based material.

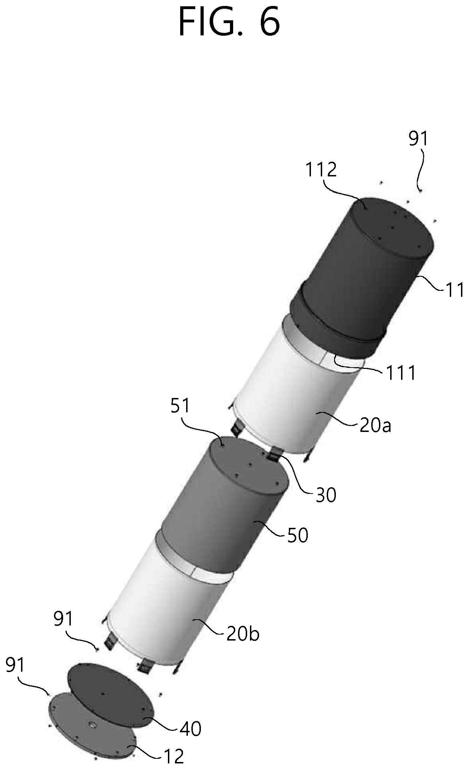

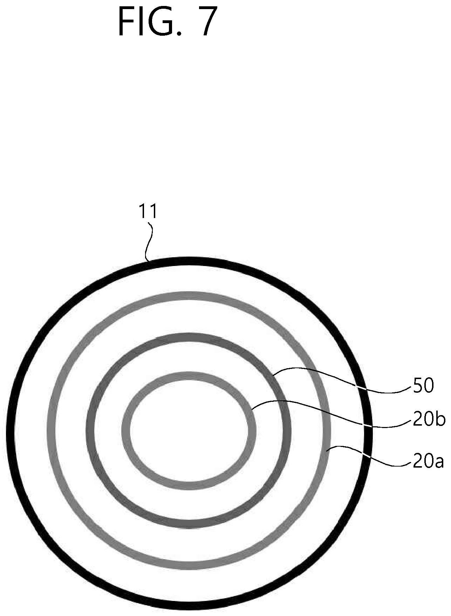

A phantom according to a second embodiment of the present disclosure will be described with reference to FIGS. 6 and 7.

FIG. 6 is an exploded perspective view of the phantom according to the second embodiment of the present disclosure, and FIG. 7 is a cross-sectional view of the phantom according to the second embodiment of the present disclosure.

Configurations different from those of the first embodiment will mainly be described below.

The phantom 1 according to the second embodiment includes a first radiation detection panel 20a and a second radiation detection panel 20b. Both the first radiation detection panel 20a and the second radiation detection panel 20b are rolled into cylindrical shapes, and as illustrated in FIG. 7, the second radiation detection panel 20b is positioned within the first radiation detection panel 20a (multi-panel layering structure).

In addition, an inner cover 50 is positioned between the first radiation detection panel 20a and the second radiation detection panel 20b to prevent interference between both panels and fix positions of the panels.

However, in FIG. 7, diameters and the like of the first radiation detection panel 20a, the second radiation detection panel 20b, and the inner cover 50 are illustrated as being similar for convenience.

In another embodiment, the first radiation detection panel 20a, the second radiation detection panel 20b, and the inner cover 50 may be disposed in close contact with each other.

Coupling holes 112 and 51 are respectively formed in the body case 11 and the inner cover 50.

According to the second embodiment, detection points of the radiation detection panels 20a and 20b are more densely arranged within the inner space.

In another embodiment, three or more radiation detection panels 20 may be provided.

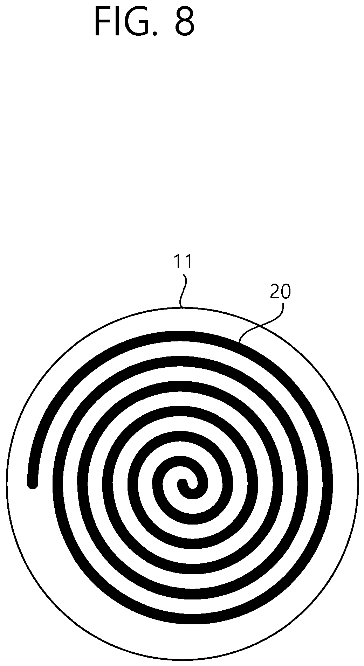

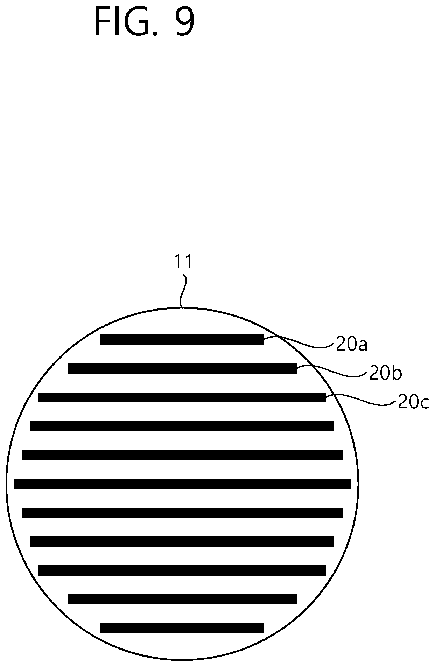

Shapes of the radiation detection panels 20 may be variously modified, which will be described with reference to FIGS. 8 and 9.

FIG. 8 is a cross-sectional view of a phantom according to a third embodiment of the present disclosure, and FIG. 9 is a cross-sectional view of a phantom according to a fourth embodiment of the present disclosure.

In the third embodiment, the radiation detection panel 20 is spirally arranged (rolled in a spiral shape). According to the third embodiment, detection points may be densely arranged within the inner space using a small number of radiation detection panels 20.

In the fourth embodiment, a plurality of flat plate-type radiation detection panels 20a, 20b, and 20c are arranged in parallel (stacked), and widths or areas of the radiation detection panels 20a, 20b, and 20c are variously provided.

In the phantom according to the present disclosure described above, detection points are three-dimensionally arranged in the inner space. Since the radiation detection panel in which multiple detection points are formed is used, it is easy to provide a large number of detection points and easily identify measurement results. In addition, when the radiation detection panel is arranged in a cylindrical shape or spiral shape, detection points may be effectively arranged in a three-dimensional space using only a small number of radiation detection panels.

The cylindrical 3D phantom according to the present disclosure is similar to a human body model, maintains a constant beam vector regardless of an angle during rotational radiation treatment, and enables 3D dosimetry.

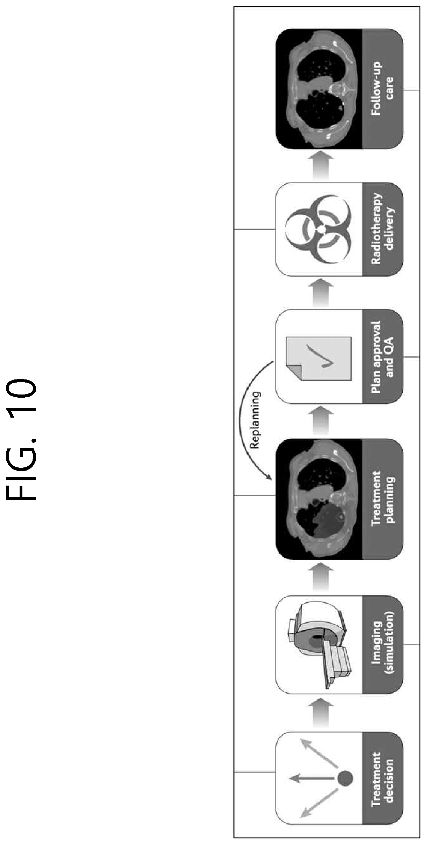

Hereinafter, a method for verifying a radiation dose using the phantom of the present disclosure will be described with reference to FIGS. 10 and 11.

Radiation treatment is a representative cancer treatment method that destroys cancer cells and stops growth thereof by using radiation emitted from a device generating high-energy radiation (X-rays, gamma rays, proton beams, and the like). Since radiation may affect both cancer cells and healthy cells, it is important to deliver a desired radiation dose to a tumor while minimizing radiation delivered to normal tissues.

As advanced radiation treatment technologies such as intensity modulated radiotherapy (IMRT) and volumetric modulated arc therapy (VMAT) have been introduced, accurate treatment is required in order to deliver a minimum dose to normal tissues around a tumor and concentrated radiation to the tumor by calculating a precise and optimized dose distribution. That is, a step of verifying a calculated dose before treatment is very important. In actual clinics, quality assurance (QA) for radiation treatment devices and patient treatment is performed.

A volumetric intensity modulated rotational treatment (VMAT) has recently been widely used because it improves treatment accuracy and minimizes radiation influence on surrounding normal tissues by using a beam rapidly rotating 360 degrees as a three-dimensional intensity-modulated radiation treatment technology. Rotational treatment techniques are possible not only in X-ray treatment but also in proton treatment.

As state-of-the-art treatment technologies require precise treatment, treatment planning and dose calculation become complicated. Comparative verification of a three-dimensional radiation dose distribution irradiated onto tumors and organs according to an anatomical structure of a patient is very important.

3D dosimetry is highly necessary to reduce errors and improve treatment effects, and the phantom according to the present disclosure may particularly be used for verifying a radiation dose of the volumetric intensity modulated rotational treatment (VMAT).

As illustrated in FIG. 10, in treatment of tumors and the like using radiation, treatment is first determined and simulation is performed. Thereafter, a treatment plan is established, and a radiation dose according to the treatment plan is verified. After verification, actual treatment and follow-up care are performed.

The phantom according to the present disclosure may be used for verification of a radiation dose in this process.

In the verification, first, the phantom is set up at a measurement position and a LAN cable is connected. Thereafter, radiation is irradiated onto the phantom including the radiation detection panel, and a radiation dose is analyzed using radiation detected by the radiation detection panel.

The phantom is filled with water or solid water to simulate a human body. In addition, since the radiation detection panel is three-dimensionally arranged in the inner space of the phantom, radiation doses at various points in the inner space may be identified.

Radiation may be irradiated 360 degrees along a circumference of the phantom, that is, along a circumference of the cylindrical inner space. Accordingly, data regarding the irradiated beam may be collected without limitation in angle. In addition, since 360-degree measurement is possible, the phantom is suitable for verification of a treatment device performing treatment while actually rotating 360 degrees.

The radiation may include, but is not limited to, at least one of X-rays, gamma rays, and proton beams.

The above-described embodiments are examples for describing the present disclosure, and the present disclosure is not limited thereto. Those skilled in the art to which the present disclosure pertains may variously modify the present disclosure based thereon. Therefore, the technical protection scope of the present disclosure should be defined by the appended claims.

## Claims

1. A phantom used for verifying a radiation dose for treatment, the phantom comprising:
an outer case forming an inner space; and
multiple detection points for detecting a dose of incident radiation,
wherein the multiple detection points include a radiation detection panel three-dimensionally arranged in the inner space and a driving circuit board positioned in the inner space and connected to the radiation detection panel.

2. The phantom of claim 1, wherein the radiation detection panel is flexible, and
the radiation detection panel is rolled in a cylindrical shape or rolled in a spiral shape.

3. The phantom of claim 2, wherein the radiation detection panel includes
an electrical insulating layer having an upper surface and a lower surface,
an upper electrode on the upper surface of the electrical insulating layer, and
a plurality of pixel units electrically connected to the electrical insulating layer and directly contacting the lower surface of the electrical insulating layer.

4. The phantom of claim 3, wherein the radiation detection panel includes
a first radiation detection panel rolled in a cylindrical shape, and
a second radiation detection panel rolled in a cylindrical shape and positioned within the first radiation detection panel.

5. The phantom of claim 4, further comprising:
an inner cover positioned between the first radiation detection panel and the second radiation detection panel,
wherein the inner cover prevents interference between the first radiation detection panel and the second radiation detection panel.

6. The phantom of claim 5, further comprising:
an integrated circuit connecting the radiation detection panel and the circuit board,
wherein the circuit board is connected to an external power line and signal line.

7. The phantom of claim 1, wherein the radiation detection panel includes
a first radiation detection panel having a flat plate shape, and
a second radiation detection panel spaced apart from the first radiation detection panel and having a width different from that of the first radiation detection panel.

8. The phantom of claim 1, wherein the inner space has a cylindrical shape.

9. A method for verifying a radiation dose, the method comprising:
irradiating radiation onto a phantom including a radiation detection panel; and
analyzing the radiation dose using the radiation detected by the radiation detection panel,
wherein the radiation detection panel includes multiple detection points for detecting a dose of incident radiation, and the multiple detection points are three-dimensionally arranged in the inner space.

10. The method of claim 9, wherein the phantom forms a cylindrical inner space,
the radiation detection panel is positioned in the inner space, and
the radiation is irradiated 360 degrees along a circumference of the inner space.

11. The method of claim 10, wherein the radiation detection panel is flexible, and
the radiation detection panel is rolled in a cylindrical shape or rolled in a spiral shape.

12. The method of claim 11, wherein the radiation detection panel includes
a first radiation detection panel rolled in a cylindrical shape, and
a second radiation detection panel rolled in a cylindrical shape and positioned within the first radiation detection panel.

13. The method of claim 11, wherein the radiation includes at least one of X-rays and proton beams.

14. The method of claim 11, wherein the verification method is used for verifying a radiation dose of volumetric intensity modulated rotational treatment.
